# EUROPEAN PATENT SPECIFICATION

(11) **EP 1 881 793 B1**
(45) Date of publication and mention of the grant of the patent: **10.07.2013**
(21) Application number: 06770326.4
(22) Date of filing: 11.05.2006
(51) Int. Cl.: A61B 17/72

(54) **BONE IMPLANT DEVICE**
KNOCHENIMPLANTATIONSVORRICHTUNG
IMPLANT OSSEUX

(30) Priority: 19.05.2005 US 682456 P; 09.05.2006 US 430752
(43) Date of publication of application: 30.01.2008
(73) Proprietor: Unigene Laboratories, Inc., Fairfield NJ 07004 (US); Yale University, New Haven, CT 06511 (US)
(72) Inventor: VIGNERY, Agnes, Branford, Connecticut 06405 (US); MEHTA, Nozer, M., Randolph, New Jersey 07869 (US); GILLIGAN, James, P., Union, New Jersey 07083 (US)
(74) Representative: Maury, Richard Philip
(86) International application number: PCT/US2006/018609
(87) International publication number: WO 2006/124708

(56) References cited:
- WO-A1-01/49193
- WO-A1-91/11148
- BE-A- 560 587
- DE-C- 742 097
- FR-A5- 2 070 264
- US-A- 4 608 052
- US-A- 5 489 306

## Description

### BACKGROUND OF THE INVENTION

### Field of the Invention

The invention relates to a bone implant device as well as to methods for its use. More particularly, the invention is directed to such a device having minimal contact with an inner surface of a bone marrow cavity wherein it is installed, while yet securely supporting, for example, another bone portion or fragment, or a prosthetic device or limb, to which the device is also connected.

### Description of the Prior Art

Bone implants are frequently inserted into the skeletal structure of humans, particularly for, but not limited to, uses such as repairing bone fracture(s) and other bone trauma, for joint (e.g., hip, shoulder, knee, ankle, etc.) replacement, and for attaching prosthetic devices and/or limb(s) to the skeleton. One major difficulty associated with the installation of such bone implants, however, is to ensure their attachment to the adjacent skeletal bone. Clearly, in those situations in which permanency is necessary or desirable, the implanted device should remain permanently adhered to the contacting bone surface. There are several common prior art methods by which such devices have been attached to the bone: (1) force-fitting the device into the medullary canal of the bone; (2) securing the device in the bone with the use of screws or pins; (3) bonding the device to the bone by the use of a "bone cement"; and (4) providing the device with a fenestration (or opening) into which bone may grow, or providing on the surface of the device a porous coating layer which serves the same purpose.

Force or friction fitting a bone implant device into the bone canal provides a very stiff overall structure capable of loosening over time. Moreover, the insertion technique often tends to damage or destroy osteoblasts located around the inner surface of the medullary canal, thus preventing or at least suppressing the very bone formation needed for securing the implant within the bone.

The use of screws and /or pins as the sole means for retaining such devices in position may lead to additional complications. Failure of a single screw or pin, e.g., while the limb containing the bone is in use following the surgery, requires a further surgical procedure to repair or entirely replace the insert since the lack of any other fixation means results in a device which is unable, under such circumstances, of performing its intended function. This is, of course, highly undesirable.

The use of bone cements creates additional difficulties. While such cements do tend to provide the initial fixation necessary to permit healing following surgery, their use typically results in a very stiff overall structure, which is often prone to loosening over time. Furthermore, their presence may provoke tissue reactions in individuals sensitive to the composition of these materials.

One example of a bone insert device comprising a fenestra or opening is shown in U.S. Patent No. 3,228,393 to Michele. The device is provided with two such openings (30,31) but it is required that the openings be packed with bone grafts (32, 33) prior to installation of the device within the bone. U.S. Patent No. 3,228,393 describes providing a portion of an insert with fenestrae. Bone grafts pass through the fenestrae and cancellous bone is packed into the remaining recesses and grooves in the fenestrae to eventually unite with the cortical bone of the grafts. Another arrangement is shown in U.S. Patent No. 6,758,849 to Michelson, which is directed to interbody spinal fusion implants. In one embodiment, the device forms a chamber defining a plurality of openings therein, wherein the chamber can be filled with and hold any natural or artificial osteoconductive, osteoinductive, osteogenic or other fusion enhancing material.

Each of the above-described methodologies requires lengthy and relatively difficult procedures in the midst of the implant operation, dealing with the introduction and packaging of foreign bone matter into the patient's bone structure. There is also the question of histocompatability of the added bone matter with the existing bone.

Implants having porous surface coatings rely for fixation on the ingrowth of bone or other connective tissue into the coating on the surface of the implant device, thereby anchoring the device to the bone. Examples of such arrangements are found in, for example, U.S. Patent No. 3,605,123 to Hahn and 5,489,306 to Gorski. A number of technical concerns are encountered with the use of such devices, however, in choosing appropriate coating materials and in determining the proper method(s) for the application of these materials to the implant device. Not all of these concerns have yet been resolved.

There has thus been a long felt need for an easily installed, yet readily secured, bone implant device providing sufficient strength for use in, e.g., repairing fractures and other bone trauma, serving as an artificial joint and acting to secure a prosthetic limb to the skeletal structure, wherein the device is configured and adapted to achieve a reduced amount of contact with the inner surface of the bone marrow cavity, i.e., the endosteum, to facilitate bone formation by osteoblasts lining the bone marrow cavity adjacent the surface of the bone implant device. The present invention is believed to adequately meet, if not surpass, each of these requirements.
WO 01/49193 and FR 2870264A disclose a bone implant device comprising a plurality of interconnected plate members configured and adapted to permit at least partial insertion of the device within an interior bone portion of a subject, said interior bone portion comprising a bone marrow cavity defined by an interior bone surface, at least one said plate member defining a plurality of apertures therein adapted for permitting bone growth therethrough from within said interior bone portion, for aiding in securing the device within the interior bone portion, wherein, following installation of the device, no more than about 75% of the interior bone surface is in contact with the device.
The invention is the device of claim 1.

The present invention can provide an apparatus for facilitating bone growth in instances including, but not limited to, the repair of bone fractures or other bone trauma. The apparatus or device possesses the dual properties of providing a rigid structure, while also providing minimal contact (as that term is defined below) with the medullary canal within the bone, thus permitting bone growth to occur with a minimal amount of impedance. The apparatus is capable, moreover, of a variety of additional uses including, but not limited to, methods for modeling bone growth in growing subjects and for anchoring prosthetic appendages to the body of a subject.

In one embodiment the invention is directed to a bone implant device according to claim 1 comprising a plurality of interconnected plate members. At least one such plate member defines a plurality of apertures adapted to permit bone growth through the aperture from an interior portion of a bone in which the device is implanted. This aids in securing the device within the interior of the bone. Moreover, the device is configured and adapted to minimize contact with the interior portion of the bone surface, thus facilitating bone growth due to osteoblasts lining the bone marrow cavity. The device is thus configured and adapted to reduce, as much as possible, contact with the inner bone surface which would otherwise serve to hinder or prevent bone formation by osteoblasts lining that bone surface, while still retaining a required amount of strength and rigidity for the device to serve its intended purpose. The need for maintaining a balance between the opportunity for growing new bone which serves to "lock" the device within the bone marrow cavity, and maintaining a required degree of strength and rigidity of the device, will be well understood by one of ordinary skill in this art.

An example of a method of using the invention for repairing a bone fracture in a subject in need of such repair comprises sufficiently stabilizing a fractured portion of a bone of the subject for a time sufficient to permit bone growth to repair the fracture, wherein the fractured portion is stabilized by inserting into a region adjacent the fractured portion a bone implant device according to the invention. The device, which is described further herein, comprises at least two interconnected plate members, wherein at least one plate member defines a plurality of apertures adapted to permit bone growth through the aperture from an interior portion of the fractured bone. This arrangement aids in securing the device within the bone. The device is configured and adapted to minimize contact with the interior surface of the bone, such that no more than 75% of the bone's inner surface is in contact with the bone's inner surface following its installation, to thus aid in preventing suppression or reduction of bone growth by osteoblasts located within the marrow cavity and/or along the inner bone surface.

Another method of using the invention is for modeling bone growth in growing subjects and it comprises causing a growing portion of at least one bone to grow into a desired shape or length. This is achieved by inserting within the bone portion a bone implant device according to claim 1. At least one plate member defines a plurality of apertures adapted to permit bone growth through the aperture from an interior portion of the bone in which the device is implanted. This aids in securing the device within the bone. The device is configured and adapted to minimize contact with the interior surface of the bone, such that no more than 75% of the bone's inner surface is in contact with the device following its installation, to aid in preventing suppression or reduction of bone growth by osteoblasts located within the marrow cavity and/or along the inner bone surface.

Another method of using the invention is for anchoring a prosthetic appendage to the body of a subject and it comprises inserting a first end portion of a bone implant device according to claim 1 within a bone stub remaining at a location where the prosthesis is to be anchored. The bone implant device comprises a plurality of interconnected plate members. At least one plate member defines a plurality of apertures adapted to permit bone growth through the aperture from an interior portion of the bone stub in which the device is implanted. The device is configured and adapted to minimize contact with the interior portion of the bone stub, such that no more than 75% of the bone's inner surface is in contact with the device following its installation, to assist in preventing suppression or reduction of bone growth by osteoblasts located within the marrow cavity of the stub and/or along the periphery of the inner bone surface. The prosthesis is secured to a second, opposed end portion of the bone implant device.

Another method of using the invention is for securing a bone implant device within an interior portion of a bone of a subject and it comprises: (a) locating at least a portion of a bone implant device according to claim 1 within a bone marrow cavity of the bone, the bone implant device comprising a plurality of interconnected plate members, wherein at least one such plate member defines a plurality of apertures adapted for permitting bone growth therethrough from an interior portion of the bone marrow cavity, for aiding in securing the device within the bone, wherein the device is configured and adapted to minimize contact with the interior portion of the bone, i.e., such that no more than 75% is in contact with the device following its installation; (b) mechanically inducing an increase in osteoblast activity in the subject; and (c) elevating the blood concentration of at least one bone anabolic agent in the subject, wherein steps (b) and (c) above are performed in any order, but in sufficient time proximity that the elevated concentration and the mechanically induced increase in osteoblast activity at least partially overlaps.

A further example of a method of using the invention involves an alternate method for securing a bone implant device within the interior bone portion and it comprises: (a) locating at least a portion of a bone implant device according to claim 1 within a bone marrow cavity of the bone, the bone implant device comprising a plurality of interconnected plate members, wherein at least one such plate member defines a plurality of apertures therein adapted for permitting bone growth therethrough from an interior portion of the marrow cavity for aiding in securing the device within the bone, wherein the device is configured and adapted to minimize contact with the interior surface of the bone, such that no more than 75% of the bone's inner surface is in contact with the device following its installation; (b) mechanically inducing an increase in osteoblast activity in the subject; and (c) administering to the subject at least one agent that causes elevated blood levels of an endogenous bone anabolic agent within the subject, wherein steps (b) and (c) above are performed in any order, but in sufficient time proximity that the elevated concentration of the anabolic agent and the mechanically induced increase in osteoblast activity at least partially overlaps.

Other features and advantages of the present invention will become apparent from the following description of the invention which refers to the accompanying drawings.

### BRIEF DESCRIPTION OF THE DRAWINGS

FIG. 1 is a sectional view of a prior art bone implant device whereby a solid rod is frictionally inserted within the bone marrow cavity;

FIG. 2 is a plan view of two slotted plate members adapted for producing, following their interconnection, a bone implant device according to one embodiment of the invention;

FIG. 3 is a sectional view through a bone having implanted therein a bone implant device according to the invention formed by interconnecting the two slotted plate members shown in Fig. 2;

FIG. 4a is a perspective view of a bone implant device formed using three plate members connected to one another via hinge members according to another embodiment of the invention;

FIG. 4b is a sectional view through a bone having implanted therein a bone implant device comprised of three plate members, illustrating one possible arrangement of the bone implant device;

FIG. 4c is a sectional view through a bone having implanted therein a bone implant device comprised of three plate members, illustrating an alternate arrangement of the bone implant device of the invention;

FIG. 5 is a sectional view through a bone having implanted therein a bone implant device comprised of four plate members according to a further embodiment of the invention;

FIG. 6a is a sectional view through a bone having implanted therein a bone implant device according to the invention comprising a centrally positioned tubular plate member having a plurality of adjustable substantially planar plate members extending therefrom;

FIG. 6b is a sectional view through a bone having implanted therein a bone implant device comprising a centrally located tubular plate member having a plurality of fixed substantially planar plate members extending therefrom;

FIG. 6c is a sectional view through a bone having implanted therein a bone implant device comprising a centrally located tubular member surrounded by a single helical plate member which extends outwardly from the central member;

FIG. 7a is a perspective view, partially in section, illustrating a bone implant device used in forming an artificial joint;

FIG. 7b is a sectional view taken along the line 7b-7b of Fig. 7a;

FIG. 8a is a perspective view, partially in section, illustrating a bone implant device used in joining a prosthetic limb to an existing bone stub; and

FIG. 8b is a sectional view taken along the line 8b-8b of Fig. 8a.

### DETAILED DESCRIPTION OF PREFERRED EMBODIMENTS OF THE INVENTION

In one embodiment the invention is directed to a bone implant device according to claim 1 comprising two or more interconnected plate members. At least one plate member defines a plurality of apertures adapted to permit bone growth through the aperture from an interior portion of a bone in which the device is implanted. The number and arrangement of these apertures is an important consideration in fabricating the plate members of the invention and the appropriate choices involving these parameters can be readily made by those having ordinary skill in this art. Bone growth through the apertures aids in securing the device within the bone marrow cavity. The device is configured and adapted to minimize contact with the interior portion of the bone to aid in preventing suppression or reduction of bone growth by osteoblasts located within the marrow cavity and/or along the inner bone surface. As used herein, the phrase minimize (or minimal) contact is defined to mean that no more than 75% of the bone's inner surface, i.e., the endosteum, is in contact with the device following its installation within the marrow cavity of a bone. In alternate embodiments of the invention, however, the degree of contact may be any percentage less than 75%. For example, the degree of contact between the device and the bone inner surface may amount to no more than 70%, 65%, 60%, 55%, 50%, 45%, 40%, 35%, 30%, 25%, 20%, 15% or 10%. These values are provided only as examples, however and should not necessarily be construed as specific limitations upon the invention, so long as the percentage of contact is no more than 75%.

The device may optionally be at least partially additionally secured within the bone marrow cavity with the use of at least one fastening device. Useful fasteners include, but are not limited to, screws, nails or clips. Alternate fastening devices capable of performing the same securing function are also considered as falling within the scope of the invention. In a further alternate embodiment, a bicompatible adhesive may be utilized to secure, or further secure, the device within the bone cavity.

The plate members of which the device is comprised are optionally interconnected at a predetermined angle to one another. The angle is chosen to correspond with the configuration of the space available within an interior bone portion, i.e., the bone marrow cavity, of a subject wherein the device is to be inserted and affixed. The chosen angular orientation may be maintained by, for example, one or more adjustment device(s) located on at least one of the plate members. Representative adjustment devices include, but are not limited to, clips, clamps, detents and the like.

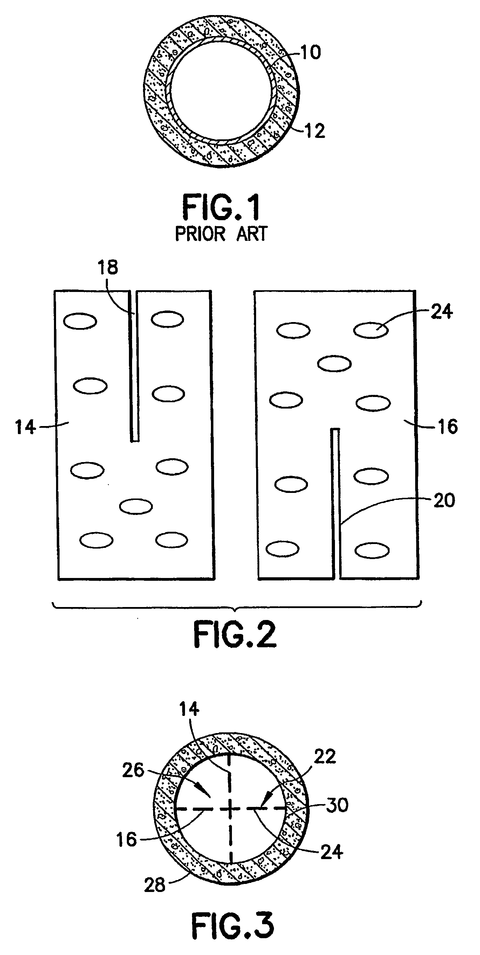

In one embodiment, one or more of the plate members define at least one slotted aperture (see, e.g., Fig. 2). The slotted aperture(s) may be configured and adapted to permit an interlocking fit between the slotted plate member and at least one additional plate member which may, if desired, be provided with a corresponding slotted aperture as well. The device, in a related embodiment, thus may be comprised of first and second interconnected plate members, wherein the plate members are interconnected by insertion of a portion of the first plate member, whether slotted or not, into a slotted aperture in the second plate member.

In an alternate arrangement, at least two plate members may be interconnected by a hinge member joining an edge portion of the plate members. Each hinge member forms an axis of rotation of the plate members connected thereby. In a related embodiment, the device may, for example, be comprised of first and second interconnected plate members, wherein the plate members are interconnected by a hinge member along an edge portion thereof, with the hinge member forming an axis of rotation of the plate members. In a still further embodiment, the device may comprise three (3), four (4), or more plate members wherein at least two of the plate members are interconnected to an adjacent member by a hinge member along an edge portion thereof, and wherein the hinge member forms an axis of rotation of the plate members which are interconnected thereby.

In one embodiment, the plates of which the device is comprised are substantially planar in shape. Alternately, at least one plate member may be at least partially curvilinear in shape. Still further, at least one said plate member may, if desired, be curved into an open tubular shape. Thus, as used herein the terms "plate" and "plate member" are not to be construed solely as referring to members having a substantially flat outer surface. That is, in accordance with the present invention one or more of the plate members used in forming the bone implant device of the invention may be at least partially curved, even to the point wherein at least one member is curved completely around so that its opposed edges come into contact with each other, thus forming an open tubular member.

In an additional embodiment of the invention, the implant device comprises at least one plate member curved into an open tubular shape, and at least one additional plate member attached at one end thereof to an outer surface of the tubular plate member, with a second, opposed end of the additional member extending outwardly from the tubular member. The at least one additional plate member extending from the tubular member is movably attached to the tubular plate member and thus may extend outwardly at an adjustable angle to the outer surface of the tubular member.

In a further embodiment of the invention, at least one plate member of the implant device may extend outwardly from an inner portion of a tubular plate member through an aperture in the tubular member, wherein the aperture through which the plate member extends is configured and adapted to permit passage of the extending plate member at that location at least partially into and out of, i.e., through, the tubular plate member. This arrangement serves to permit adjustment of the distance the extending member extends outwardly from the tubular plate member to take into account, for example, bones having relatively small or irregularly shaped marrow cavities, thus permitting a customized fit of the implant device therein. The outwardly extending plate member may, if desired, be provided with an adjustment mechanism for controlling and/or adjusting the distance the extending plate member extends outwardly from the tubular plate member. The adjustment mechanism may comprise, for example, at least one detent or clip.

A tubular plate member of a bone implant device can be provided along its outer surface with at least one outwardly extending member having a helical configuration. The helical member may be formed integral with or else be detachably secured to the tubular plate member. The tubular plate member can be provided with a single, continuous outwardly extending plate member on its outer surface, wherein the outer plate member has a helical configuration. This configuration is preferable in that it provides what is believed to be the greatest amount of rigidity to the bone in which it is inserted, corresponding with the least practical amount of contact between the device and the bone.

The plate members of the invention may be formed of a variety of materials including, but not limited to, various metals, ceramics, plastics, carbon composites and resins. Useful metals include, but are not limited to, stainless steel, titanium and alloys of cobalt and chrome. In one embodiment, the plates are formed of a strong, durable, non-rusting metal, such as titanium. A non-exclusive example of a useful plastic is polyethylene. Various combinations of materials may also be used in forming devices according to the invention including, as a non-limiting example, composites comprising one or more ceramics combined with one or more plastic polymers. One non-limiting example of such a composite is sold under the name PLASTI-BONE® by Advanced Ceramic Research, Inc. located in Tucson, Arizona U.S.A. In choosing an appropriate material of which to form the device of the invention, it is important to keep in mind that the material must be bio-compatible, as well as resistant to corrosion, degredation and wear. In an additional embodiment of the invention, however, the device may be formed of a biodegradable material, chosen or adapted to biodegrade after a predetermined length of time, e.g., the time needed for repair of a fracture in a fractured bone. The gradual breakdown or elimination of the device would also serve, moreover, to provide additional room within the marrow cavity for further bone growth. In further embodiments, the device could be formed, for example, of an osteo-conductive material, adapted to direct the formation of bone in, e.g., a predetermined direction, and/or an osteo-inductive material adapted to induce, e.g., additional and/or faster, bone formation. Still further, the device could be comprised of elements formed from more than one of the materials described above, i.e., a biodegradable material, an osteo-conductive material and/or an osteo-inductive material. One of ordinary skill in this field of art would be readily able to determine the proper material for use in forming the plate members of which the device is comprised.

In an additional embodiment at least one plate member is at least partially coated, or at least partially impregnated, with a bone anabolic agent for promoting bone growth within the interior portion of the bone within which the device is implanted. The bone anabolic agent may be, but is not necessarily, selected from the group consisting of a parathyroid hormone (PTH) or truncate thereof, in the free acid or amide form, anabolic Vitamin D analogs, a low-density lipoprotein receptor-related protein 5 (LRP5), an activator of non-genomic estrogen-like signaling (ANGELS), a bone morphogenic protein (BMP), an insulin-like growth factor (IGF), a fibroblast growth factor (FGF), sclerostin, leptin, a prostaglandin, a statin, strontium, a growth hormone, a growth hormone releasing factor (GHRF), hepatocyte growth factor (HGF), calcitonin gene related peptide (CGRP), parathyroid hormone related peptide (PTHrP), Transforming Growth Factor (TGF)-β1 and combinations thereof. Alternately, the growth factors described above can be used to coat the surface of the device by a technique known as Surface Induced Mineralization (see, e.g., Voelker, JAMA (1998), vol. 280, p. 315, incorporated herein by reference).

In a particular embodiment of the invention the bone anabolic agent may be at least one parathyroid hormone. The parathyroid hormone may be one selected from the group consisting of natural parathyroid hormone, truncated natural parathyroid hormone in the free acid form, an amidated truncate of natural parathyroid hormone and combinations thereof. Truncates of natural parathyroid hormone useful with the invention include, but are not limited to, PTH[1-30], PTH[1-31], PTH[1-32], PTH[1-33], and PTH[1-34], in the free acid or amide form, and combinations thereof. PTH[1-34]OH and PTH[1-34]NH₂ have been found to be particularly useful in the invention.

In an alternate embodiment at least one plate member of the bone implant device of the invention is at least partially coated, or at least partially impregnated with an agent that causes an increased expression of an endogenous bone anabolic agent into the blood of a subject within which the device is implanted. In one embodiment the at least one plate member is at least partially coated or impregnated with a calcilytic agent applied in therapeutically useful amounts. Alternately, however, if desired, the bone anabolic agent and/or an agent causing increased expression of endogenous bone anabolic agent may be directly administered, in a systemic fashion, to the patient, rather than being coated on or impregnated into, the plate members. The methodologies, dosages, etc. relating to such administration are as described below in the discussion of an embodiment of the invention incorporating the mechanical inducement of osteoblast activity.

The number and arrangement of the apertures adapted for permitting bone growth, i.e., to "lock" the implant device within the bone, may vary widely depending upon the intended application for the device. Either or both the number of apertures and/or their distribution upon one or more plate members of the device may be altered as necessary to take into account a particular use to which the device is to be put. There is, however, a well-understood trade off between aperture volume (i.e., empty space) and plate member strength. That is, increasing the amount of empty space defined by a plate member by, for example, increasing the number and/or size of the apertures, will likely result in a loss of plate strength unless a corrective measure, such as thickening the web portion of the plate or forming the plate member from a material having an enhanced weight-bearing capability and/or resistance to stresses induced by twisting, etc., is undertaken so as to permit a subject to carry out their normal everyday activities. Additionally, the device must possess sufficient strength to permit it to be wedged, without damage thereto, within a subject's bone marrow cavity during installation. As a non-limiting guideline, the device should possess the following degree of compressive strength (as measured in MegaPascals, i.e., MPa's) depending upon the bone in which it is to be installed: (1) in the femur, the device should provide a compressive strength of at least about 167 MPa; (2) in the humerus, at least about 132 MPA; (3) in the radius, at least about 114 MPA; (4) in the tibia, at least about 159 MPA; (5) in the neck, at least about 10 MPA; and (6) in the lumbar vertebrae, at least about 5 MPa.

The accompanying drawings, which are incorporated into and constitute part of, this specification, illustrate a variety of features and embodiments of the bone implant device according to the invention and, together with the description provided herein, they serve to explain the principles of the invention. It is to be understood, of course, that both the drawings and the description are explanatory only and are not restrictive of the invention.

Fig. 1 provides, for comparison purposes with the present invention (as shown in the remaining figures), an illustration of a bone implant device constructed according to the prior art wherein the device comprises a substantially solid rod member 10, which is force or friction fit into the medullary cavity (not shown) of a bone 12. As noted above, prior art devices of this type suffer from a variety of inadequacies, not least of which is the tendency of the device to loosen upon use of, for example, a limb incorporating the bone following implantation of the device, and the damage such frictional insertion typically causes to the osteoblasts remaining in the medullary canal when the solid rod is jammed therein. The reduction or total destruction of such osteoblasts thus significantly decreases the amount of bone formed around the outer periphery of the implant, which bone growth is vitally important for maintaining the implant in position within the bone cavity.

In Fig. 2 is shown two substantially planar plate members 14, 16 which may be interconnected via slotted apertures 18, 20 to form bone implant device 22 as shown in Fig. 3. Plate members 14 and 16 are each provided with a plurality of apertures 24. Apertures 24 are configured and adapted to permit bone produced by osteoblasts remaining in the medullary cavity upon installation of the device to grow therethrough to the opposed side(s) of the plate member(s), thus locking device 22 within the bone interior. Moreover, the width of slotted apertures 18, 20 may be varied as desired to provide a degree of "play" in the angle between plate members 14 and 16. This is to enable the surgeon installing the device to take into account the amount and configuration of the space available within the inner, i.e., bone marrow, cavity 26 of bone 28 for installation of device 22.

Fig. 3 shows device 22 implanted within the cavity 26 of a bone 28. As may be clearly seen in, for example, Fig. 3, device 22 is configured and adapted to minimize (i.e., no more than 75%) contact between plates 14,16 comprising the device and the interior surface 30 of bone 28. This permits osteoblasts located adjacent surface 30 to be subsequently converted to bone which grows through apertures 24, thus locking device 22 in place within bone 28

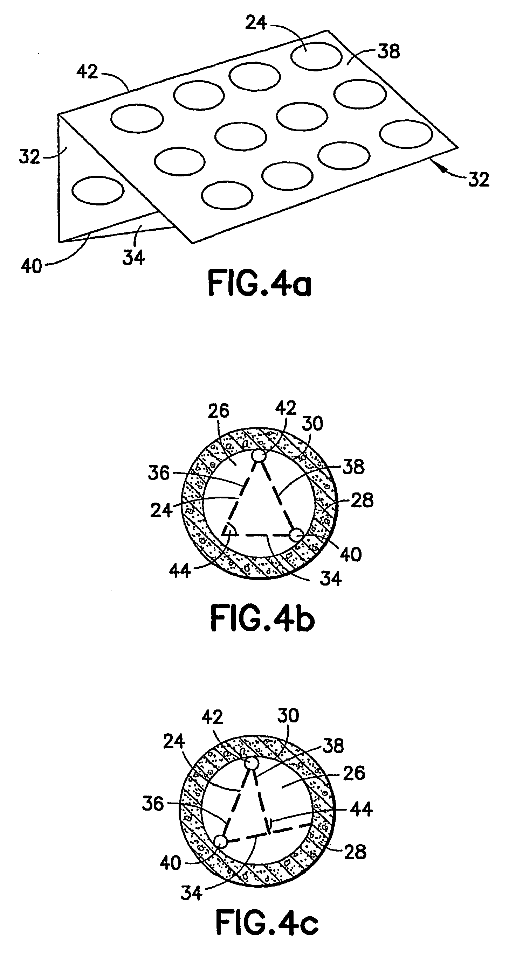

Fig. 4a shows a bone implant device 32 produced according to the invention wherein device 32 is comprised of three substantially planar plate members 34, 36,38 substantially similar in appearance to plate members 14, 16 shown in Fig. 2, except that slotted apertures 18, 20 may optionally be dispensed with as plate members 34, 36, 38 are joined together by hinge members 40,42 located along their opposed longitudinal edges. Hinge members 40, 42 form an axis of rotation for plate members 34,36,38. At least one said plate member is provided with one or more aperture(s) 24 which serve, as noted above in the discussion of Fig. 2, to permit bone growth therethrough which serves to lock device 32 within a bone in which it is installed.

Fig. 4b illustrates an embodiment wherein device 32 is installed within bone cavity 26 in a triangular orientation, providing a great deal of strength to device 32 while minimizing contact between device 32 and inner bone surface 30. The plate members may optionally be rigidly connected but, as shown, they also may be connected by hinge members 40, 42 to permit relative movement of each plate member toward and away from the other plate members. A device such as a clip or a detent 44 may be utilized to maintain plate members 34,36,38 in a desired orientation.

Fig. 4c illustrates a variation in the arrangement shown in Fig. 4b, wherein one "leg" of the triangle, formed by plate member 38, is rotated slightly inwardly toward plate member 36 via hinge member 42 and maintained in the indicated position through the use of, e.g., a clip or detent 44.

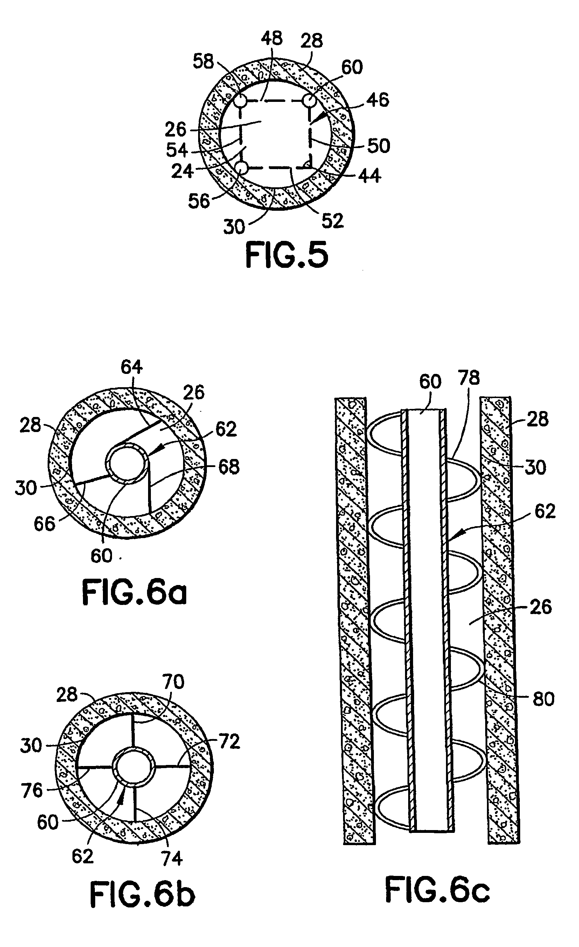

Fig. 5 illustrates a further variation of the invention wherein a bone implant device 46 is formed of four plate members 48,50,52,54. The plate members may, if desired, be rigidly connected. As shown, however, plate members 52,54 are connected via hinge member 56; plate members 54 and 48 via connected hinge member 58; and plate members 48 and 50 are connected via hinge member 60.

Figs. 6a illustrates an alternate embodiment of the invention comprised of a central tubular shaped plate member 60 defining a plurality of apertures24 (not shown) therein. Extending from the outer surface of tubular member 60 is one or more plate member(s) configured and adapted for contacting the inner surface 30 of bone 28 within which the bone implant device 62 of the invention is implanted. Fig. 6a illustrates one variation provided with three outwardly extending substantially planar plate members 64,66,68, one or more of which defines a plurality of apertures 24 (not shown) configured and adapted to permit bone growth therethrough for locking device 62 within bone cavity 26. Plate members 64,66, 68 may be made adjustable in that the angle between each plate member and the outer surface of tubular plate member 60 may be modified as required depending upon the amount and configuration of the space available within bone cavity 26. Still further, if desired, slots (not shown) may be provided in tubular member 60 such that plate members 64,66, 68 extend outwardly from these slots and may thus be moved inwardly or outwardly, i.e., toward or away from tubular plate member 60, respectively, as required, in order to customize the "fit" of device 62 within cavity 26. Once the proper degree of extension is determined, these moveable plates may, in one embodiment, be locked into position.

The implant device shown in Fig. 6b is similar in most respects to that shown in Fig. 6a, except that the implant device in Fig. 6b comprises four outwardly extending plate members 70,72,74,76. These four plate members may be rigidly or adjustably connected to tubular plate member 60 as described above with regard to Fig. 6a, and at least one said plate member 70,72, 74, 76 and preferably all said members, is provided with apertures 24 (not shown) configured and adapted for the purpose described above.

In the implant device shown in Fig. 6c, the device 78 comprises a tubular plate member 60 centrally located within bone cavity 26 and at least partially surrounded by a single helically-shaped plate member 80 which winds around the outer surface of tubular plate member 60. Optionally, helical plate member 80 is also provided with apertures 24 (not shown) for permitting bone growth therethrough.

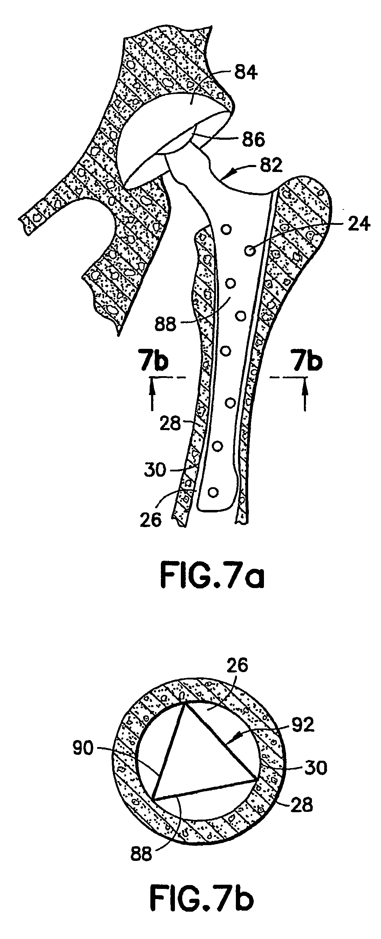

Figs. 7a,b are directed to an implant device 82 used in forming an artificial joint. The implant device will work equally effectively upon use in forming hip joints, as well as joints other than hip joints, such as knee joints, shoulder joints and the like. Thus, it is only for the purpose of illustration that the artificial joint shown is a hip joint. Device 82 is comprised of acetabular head member 84 which is pivotally connected, as at 86, to a substantially triangularly-shaped structure comprised of three plate members 88,90,82 (only plate member 88 can be seen in the view shown in the Figure) which is inserted within cavity 26 formed in bone 28. At least one, and preferably all three, plate members 88,90,92 define a plurality of apertures 24 configured and adapted to permit bone growth due to osteoblasts located along the inner surface 30 of bone 28 to pass into an inner open triangular portion defined by plate members 88,90,92 of device 82.

Fig. 7b provides a cross-sectional view of device 82 taken along line 7b-7b of Fig. 7a illustrating the triangular arrangement of plate members 88,92,92. As can be seen from the figure, such a triangular arrangement minimizes contact between device 82 and the inner surface 30 of femur bone 28 in that only the "points" of the triangular shape actually contact surface 30 of the bone.

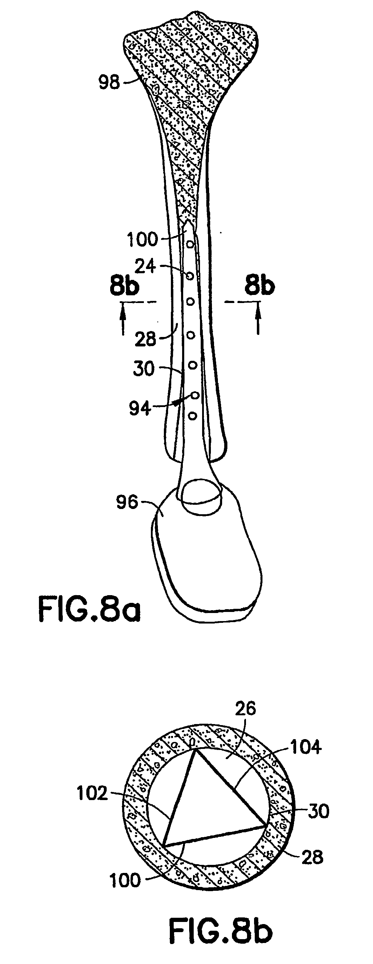

Figs. 8a, b are directed to a bone insert device 94 used in joining a prosthetic limb 96 to an existing bone stub 98. Device 94 is comprised of three co-joined plate members 100,102,104 (only plate member 100 can be seen in Fig. 8a), one or more of which defines a plurality of apertures 24 as described above.

Fig. 8b provides a cross-sectional view of device 94 taken along line 8b-8b of Fig. 8a illustrating the triangular arrangement of plate members 100,102,104. As in the case in Figs. 7a,b, this arrangement minimizes (no more than 75%) contact between device 94 and the inner surface 30 of bone 28.

In accordance with the description contained herein, it is important to note that the device 82 of Figs. 7a,b and the device 94 of Figs 8a,b are not limited to a triangular-shaped body formed of three plate members defining a plurality of apertures configured and adapted for permitting bone growth therethrough. That is, the devices 82, 94 illustrated therein may optionally be formed using alternate arrangements, such as those illustrated in Figs. 5 and 6a, b, c for example.

The invention can be used in a method for repairing a bone fracture in a subject in need of such repair. The method comprises sufficiently stabilizing a fractured portion of a bone of the subject for a time sufficient to permit bone growth to repair the fracture, wherein the fractured portion is stabilized by inserting into a region adjacent the fractured portion a bone implant device as described herein.

The invention can be used in a method for modeling bone growth in growing subjects. The method comprises causing a growing portion of at least one bone to grow into a desired shape or length. This is achieved by inserting within the bone portion a bone implant device as described herein.

The invention can be used in a method for anchoring a prosthetic appendage to the body of a subject. The method comprises inserting a first end portion of a bone implant device as described herein within a bone stub remaining at a location where the prosthesis is to be anchored, and wherein the prosthesis is secured to a second, opposed end portion of the bone implant device.

The bone implant device used in practicing the methods, including the repair of bone fractures, modeling bone growth in growing subjects and anchoring a prosthetic appendage to a subject's body, is according to claim 1. The usefulness of the device is not limited solely to the indicated methods, however, since the skilled artisan may determine a variety of additional uses for the device according to the invention.

Installation of a bone implant device according to the invention within a subject can be coupled with a treatment of the subject which induces greater and more rapid bone formation than would otherwise normally occur, which bone formation serves, as indicated above, to secure, i.e., anchor, the bone implant device within the bone of the subject. A variety of methods are well-known in the art for fostering such bone formation. However, such methods are typically systemic in nature. That is, they treat the whole skeleton as a single entity. Certain of the methods described herein, however, permit the targeting of one or more specific bones, e.g., those of the hip, shoulder, spine or wrist, which may require a more focused treatment.

The treatment can induce bone formation in a subject by a method which comprises the steps of (a) mechanically inducing an increase in osteoblast activity in the subject; and (b) elevating blood concentration of at least one bone anabolic agent therein, e.g., by administering such an agent or by administering a compound which causes natural formation of such an agent. Typically, the inducement step occurs prior to, or concurrent with, installation of the bone implant device. Inducement of bone growth, as that phrase is used herein, may include, for example, generating new or additional bone at locations where such bone growth is not presently taking place and/or stimulating the growth (i.e., increasing the rapidity thereof) of bone which is already in the process of formation. Administration of the bone anabolic agent may commence, if desired, prior to such installation and typically continues for a pre-determined period beyond the installation step. Steps (a) and (b) above may be performed in any order, but are to be carried out in sufficient time proximity that the elevated concentration of the anabolic agent and the mechanically induced increase in osteoblast activity at least partially overlaps. Therefore, the bone anabolic agent can be administered to the subject contemporaneous with the mechanical inducement of osteoblast activity. The bone anabolic agent can alternatively be administered subsequent to such mechanical inducement. The bone anabolic agent may as well be administered prior to the mechanical inducement such that elevated levels of bone anabolic agent are already present at the time of mechanical inducement, which levels may then be maintained or continued intermittently for an extended period thereafter. Without being bound in any way by theory, applicants believe that the inducement of bone growth takes place due to the combined effects of (1) the mechanical inducement of osteoblast activity in the subject, coupled with (2) an elevation in the blood concentration of the at least one bone anabolic agent.

The bone in which the bone implant device of the invention is installed defines a bone marrow cavity therein. The bone marrow cavity contains, *inter alia,* a quantity of bone marrow and a plurality of osteoblasts. The method thus comprises mechanically altering the contents of the bone marrow cavity to thereby stimulate and thus increase osteoblast differentiation and/or activity therein. Thereafter, bone mass is increased within the cavity due to the increased osteoblast differentiation/activity. The method additionally comprises administering to the subject at least one bone anabolic agent for a duration and at a concentration sufficient to raise blood levels of the anabolic agent within the subject above natural levels thereof, and thereby prolonging the mechanically induced osteoblast activity. The mechanical alteration of the bone marrow cavity thus permits specific bone(s) of the subject, i.e., those within which the bone implant device is implanted, as well as bone(s) and/or fragment(s) located adjacent thereto, to be specifically targeted for inducing bone formation therein.

Mechanical inducement of an increase in osteoblast activity may be obtained by a process of bone marrow irrigation and ablation. Again, without being bound in any way by theory, applicants believe that the bone marrow irrigation and ablation process leads to the formation of a clot within the bone marrow cavity which, through a cascade of biochemical reactions, contributes to increasing osteoblast activity in the subject.

The increased osteoblast activity may alternately be obtained by coupling the mechanical inducement with an additional form of inducement, such as biochemical inducement. Such biochemical inducement may be obtained by administering to the subject, for example, a quantity of a blood factor such as Factor ("F") VII, Factor VIIa or a combination thereof. Following tissue or vascular injury, clotting is initiated by the binding of plasma FVII/FVIIa to tissue factor (tissue thromboplastin). This complex (FVII/FVIIa + Thromboplastin) initiates a sequence of events which leads to activation of the coagulation cascade, ultimately leading to fibrin deposition and platelet activation. This complex sequence of events may contribute in part to the stimulation of osteoblasts in the bone marrow. Factors VII and VIIa are commercially available from Novo Nordisk.

The increase in osteoblast activity obtained with the use of the method above may be due to a variety of factors including, but not necessarily limited to (1) osteoblast differentiation, i.e., the production of additional osteoblasts, (2) increasing the activity and/or effectiveness of osteoblasts which are already present in inducing bone formation in the subject, and (3) a combination thereof. The increase in osteoblast activity would preferably include all of the above-noted functions.

A bone anabolic agent endogenously produced in the human body is PTH[1-84] in the free acid form which is naturally found in levels of less than about 8 picomoles (pmoles) per liter in the blood of a human subject. In the method described above involving administration of a material causing an increased expression (i.e., above the natural level described above) of an endogenous bone anabolic agent, the material administered may, for example, be a calcilytic agent. Calcilytic agents useful with the method include, but are not limited to any agent that limits the binding of calcium to its receptor and thereby triggers the release of endogenous PTH. Examples of such calcilytic compounds are set forth in United States Patents Nos. 6,362,231; 6,395,919; 6,432,656 and 6,521,667, the contents of which are incorporated herein by reference.

Both bone anabolic agents and agents causing an increased expression of bone anabolic agents, may be administered, for example, orally, intravenously, intramuscularly, subcutaneously, via implant, transmucosally, transdermally, rectally, nasally, by depot injection, or by inhalation and pulmonary absorption. Either medicament may be administered once as a time release formulation, a plurality of times or over one or more extended periods. It is preferred that elevated blood levels of the anabolic agent be maintained, at least intermittently, for between 14-365 days, and more preferably for between about 30-180 days, post-mechanical induction. Intermittent administration of a parathyroid hormone, e.g., PTH[1-34]NH₂, could occur once daily or once weekly, resulting in peaks of blood concentration that return to baseline levels between doses, but nevertheless result in periodic elevated blood levels of a bone anabolic agent in a manner that overlaps the elevated osteoblast activity that is initially induced mechanically, although thereafter sustained, at least in part, by the anabolic agent.

As in the description provided above, anabolic agents useful with the invention include, but are not limited to, a parathyroid hormone (PTH) or truncate thereof, in free acid or amide form, anabolic Vitamin D analogs, a low-density lipoprotein receptor-related protein 5 (LRP5), an activator of non-genomic estrogen-like signaling (ANGELS), a bone morphogenic protein (BMP), an insulin-like growth factor (IGF), a fibroblast growth factor (FGF), sclerostin, leptin, a prostaglandin, a statin, strontium, a growth hormone, a growth hormone releasing factor (GHRF), hepatocyte growth factor (HGF), calcitonin gene related peptide (CGRP), parathyroid hormone related peptide (PTHrP) and combinations thereof. As used herein the term "parathyroid hormone" includes, but is not limited to natural parathyroid hormone, a truncate of natural parathyroid hormone, an amidated truncate of natural parathyroid hormone, an amidated natural parathyroid hormone and combinations thereof.

The bone anabolic agent can be truncated PTH[1-34] in the free acid form. This material is commercially available in an FDA-approved pharmaceutical formulation from Eli Lilly & Co. under the trade name Forteo® (teriparatide). Other desirable bone anabolic agents for use with the invention include, but are not limited to, the amidated truncates of the natural parathyroid hormones noted above, i.e., PTH[1-30], PTH[1-31], PTH[1-33], in the free acid or the amide form, PTH[1-34]NH₂ and combinations thereof. The bone anabolic agent can preferably be PTH[1-34]NH₂. for the preparation of truncated parathyroid hormones are described in U.S. Patent No. 6,103,495 to Mehta et al. Moreover, methodologies for amidating such truncated parathyroid hormones are provided in, for example, U.S. Patents 5,789,234 to Bertelsen et al. and 6,319,685 to Gilligan et al.

A sufficient amount of amidated truncated parathyroid hormone as discussed herein can be administered to a subject to achieve, and thereafter maintain, a pulsatile blood concentration thereof in the subject of between abut 50 and 350 pg/ml, preferably between about 100 and 200 pg/ml, and most preferably about 150 pg/ml. The blood concentration of the parathyroid hormone in the subject can alternatively be raised to preferred level in no later than 7 days following mechanical alteration of the contents of the bone marrow cavity. As would be well known in this art, an appropriate dosage of the PTH bone anabolic agent must be calculated to achieve the above-indicated blood concentrations. In the case of injectable formulations, for example, the dose (in pure weight of active hormone) given to, for example, a human subject, may be that taught in the literature relating to the bone anabolic activity of these various agents. Such dose may, but does not necessarily, range between about 100-200 *µ*g, given once per day, more preferably between about 20-100 *µ*g per dose and most preferably between about 20 -50 *µ*g per dose. For alternate delivery routes, i.e., other than injections, the dosage may range between about 10*µ*g and 10mg. Dosage levels of injectable formulations comprising bone anabolic agents other than the above-described parathyroid hormone-based agents would be consistent with those noted above for the PTH agents.

The method can additionally comprise providing the subject with an elevated blood concentration of at least one antiresorptive agent, wherein the elevated concentration is sufficient to diminish resorption of new bone growth produced due to the mechanically induced enhanced osteoblast activity described above. The antiresorptive agent may be administered contemporaneous with the administration of the bone anabolic agent. The antiresorptive agent can alternatively be administered subsequent to the administration of the bone anabolic agent. The administration of the antiresorptive agent may alternatively be commenced during administration of the bone anabolic agent and such administration may then be continued beyond the termination of administration of the bone anabolic agent. Administration of the antiresorptive agent preferably continues for at least three months and more preferably between 12-24 months.

A single agent may as well be administered having both bone anabolic and antiresorptive properties. Examples of such materials include, but are not limited to, estrogen, strontium ranalate and selective estrogen receptor modulators (SERMS).

The antiresorptive agent may be a calcitonin selected from the group consisting of human calcitonin, salmon calcitonin ("sCT"), eel calcitonin, porcine calcitonin, chicken calcitonin, calcitonin gene related peptide (CGRP) and combinations thereof. The antiresorptive agent is preferably salmon calcitonin. Blood levels of calcitonin, when used as the antiresoprtion agent, preferably range between about 5-500 pg/ml, more preferably between about 10-250 pg/ml and most preferably between about 20-50 pg/ml. Moreover, human dosage levels of the subject calcitonin agents necessary to achieve the above blood levels, in the case of, e.g., injectable formulations, may be those taught in the literature relating to the use of these materials as anabolic agents. Such dose may, but does not necessarily, range between about 5-200 µg given once per day, more preferably between about 5-50 *µ*g and most preferably 8-20 *µ*g by weight of the pure drug, administered daily. When using alternate delivery routes, the range may vary between about 5*µ*g and 5mg. Salmon calcitonin (sCT) administered by alternate routes, i.e., by nasal or oral administration, would require higher dosages than those discussed above.

Alternately, a variety of antiresorptive agents other than the calcitonins are useful in the present invention. These include, generally, hormone replacement therapy (HRT) agents, such as selective estrogen receptor modulators (SERMS), bisphosphonates, cathepsin-K inhibitors, strontium ranalate and various combinations thereof. Specific examples of additional antiresorptive agents include, but are not limited to, Premarin® available from Wyeth Laboratories, which includes estrogen as the active ingredient - a typical accepted dosage is one 0.625 mg tablet daily; (2) Actonel® available from Proctor & Gamble, which includes, as the active ingredient, risedronate sodium. A typical accepted dosage is one 5 mg tablet daily or one 35 mg tablet weekly; (3) Evista® sold by Eli Lilly & Co., which includes raloxifene HCl as the active ingredient. A typical accepted dosage of this formulation is one 60 mg tablet taken daily; and (4) Fosamax® available from Merck Pharmaceuticals, which includes alendronate as the active ingredient. Typical dosages of this material are 10 mg/day or 70 mg/week.

Except where otherwise noted or where apparent from the context, dosages herein refer to the weight of the active compounds unaffected by pharmaceutical excipients, diluents, carriers or other ingredients, although such other ingredients are typically included in the variety of dosage forms useful in the invention. Any dosage form (i.e., capsule, tablet, injection or the like) commonly used in the pharmaceutical industry is appropriate for use herein and the terms "excipient", "diluent" and "carrier" include such non-active ingredients as are typically included, together with active ingredients, in the industry. For example, typical capsules, pills, enteric coatings, solid or liquid diluents or excipients, flavorants, preservatives and the like are included. Moreover, it is additionally noted that with respect to all of the dosages recommended herein, the attending clinician should monitor individual patient response, and adjust the dosages accordingly.

The antiresorptive agent may be administered orally, intravenously, intramuscularly, subcutaneously, via implant, transmucosally, rectally, nasally, by depot injection, by inhalation and pulmonary absorption or transdermally. Moreover, the antiresorptive agent may be administered once, a plurality of times, or over one or more extended periods.

A kit containing the above-described elements can be provided i.e., one or more bone implant device(s), at least one container having therein at least one bone anabolic agent and a mechanical alteration device for altering contents of a bone marrow cavity in at least one bone in which the device is to be installed. The kit may additionally comprise an evacuation device for evacuating at least a portion of the contents from the bone marrow cavity. The kit may further comprise at least one container having therein at least one antiresorptive agent.

In one arrangement of the kit, the bone anabolic agent is selected from among natural parathyroid hormone, a truncate of natural parathyroid hormone, an amidated natural parathyroid hormone, and combinations thereof. The bone anabolic agent is a truncate of natural parathyroid hormone. A preferred truncate for use as the agent is PTH[1-34] in the free acid form. Other preferred truncates include amidated truncates. The bone anabolic agent may, in such a case, be thus selected from among PTH[1-30]NH₂, PTH[1-31]NH₂, PTH[1-32]NH₂, PTH[1-33]NH₂, PTH[1-34]NH₂ and combinations thereof. In a specific embodiment, the bone anabolic agent is PTH[1-34]NH₂.

The antiresorptive agent can be a calcitonin selected from the group consisting of human calcitonin, salmon calcitonin, eel calcitonin, elkatonin, porcine calcitonin, chicken calcitonin, calcitonin related gene peptide (CRGP) and combinations thereof. The antiresorptive agent can be salmon calcitonin.

The present invention is limited only by the appended claims.

## Claims

1. A bone implant device (22) comprising a plurality of interconnected plate members (14, 16) configured and adapted to permit at least partial insertion of the device (22) within an interior bone portion of a subject, said interior bone portion comprising a bone marrow cavity (26) defined by an interior bone surface (30), at least one said plate member (14, 16) defining a plurality of apertures (24) therein adapted for permitting bone growth therethrough from within said interior bone portion, for aiding in securing the device (22) within the interior bone portion, wherein, following installation of the device (22), no more than about 75% of the interior bone surface (30) is in contact with the device (22), and **characterized in that** an angle between at least two of said interconnected plate members (14, 16) is adjustable.

2. The implant device (22) according to claim 1, wherein the device is at least partially secured within said interior bone portion with at least one fastener or with a biocompatible adhesive.

3. The implant device (22) according to claim 1, wherein said plate members (14, 16) are maintained at a predetermined angle to one another, said angle chosen to correspond with an amount and a configuration of a space available within said interior bone portion for implanting the device.

4. The implant device (22) according to claim 3, wherein said predetermined angle is maintained by at least one adjustment device (44) located on at least one said plate member, said adjustment device (44) being selected from the group consisting of clips, clamps and detents.

5. The implant device (22) according to claim 1, wherein at least one said plate member (14, 16) defines a slotted aperture (18, 20) therein, said slotted aperture being configured and adapted to permit an interlocking fit between said slotted plate member and at least one additional plate member.

6. The implant device (22) according to claim 1, wherein at least two said plate members (14, 16) are connected by a hinge member (40, 42) along an edge portion of the plate members thus connected, each said hinge member (40, 42) forming an axis of rotation of the plate members connected thereby.

7. The implant device (22) according to claim 1, comprising three or more said plate members (34, 36, 38), wherein at least two said plate members arc connected to an adjacent member by a hinge member (40, 42) along an edge portion of the connected plate members, each said hinge member forming an axis of rotation of the plate members interconnected thereby.

8. The implant device (22) according to claim 1, wherein said plate members (14, 16) have a shape which is selected from the group consisting of substantially planar, at least partially curvilinear, and open tubular.

9. The implant device (22) according to claim 8, wherein said plate member (60) has an open tubular shape and is provided with at least one additional plate member (64, 66, 68) attached at one end thereof to an outer surface of said tubular plate member and having a second, opposed end extending outwardly from said tubular plate member.

10. The implant device (22) according to claim 9, wherein at least one said additional plate member (64, 66, 68) extending from said tubular plate member (60) extends at an adjustable angle to an outer surface of said tubular plate member.

11. The implant device (22) according to claim 1, wherein said plate members (14, 16) are formed of a material selected from the group consisting of metals, ceramics, plastics, composites and resins.

12. The implant device (22) according to claim 1, wherein at least one said plate member (14, 16) is at least partially coated or at least partially impregnated with a bone anabolic agent for promoting bone growth within the interior portion of a bone wherein the device (22) is implanted.

13. The implant device (22) according to claim 12, wherein the bone anabolic agent is selected from the group consisting of a parathyroid hormone (PTH) or truncate thereof, in free acid or amide form, anabolic Vitamin D analogs, a low-density lipoprotein receptor-related protein 5 (LRP5), an activator of non-genomic estrogen-like signaling (ANGELS), a bone morphogenic protein (BMP), an insulin-like growth factor (IGF), a fibroblast growth factor (FGF), sclerostin, leptin, a prostaglandin, a statin, strontium, a growth hormone, a growth hormone releasing factor (GHRF), heptocyte growth factor (HGF), calcitonin gene related peptide (CGRP), parathyroid hormone related peptide (PTHrP), transforming growth factor (TGF)-β1 and combinations thereof.

14. The implant device (22) according to claim 12, wherein the bone anabolic agent is selected from the group consisting of PTH[1-30], PTH[1-31], PTH[1-32], PTH[1-33], and PTH[I-34], in the free acid or amide form, and combinations thereof.

15. The implant device (22) according to claim 1, wherein at least one said plate member (14, 16) is at least partially coated or at least partially impregnated with an agent that causes increased expression of an endogenous bone anabolic agent into the blood of a subject within which said device (22) is implanted.

16. The bone implant device (22) of claim 1, wherein at least one said plate member (14, 16) defines a slotted aperture (18, 20) therein, said slotted aperture being configured and adapted to permit an interlocking fit between said slotted plate member and at least one additional plate member, said plate members having a shape selected from the group consisting of substantially planar, at least partially curvilinear and open tubular and being formed of a material selected from the group consisting of metals, ceramics, plastics, composites and resins, wherein said plate members are maintained at a predetermined angle to one another, said angle chosen to correspond to an amount and a configuration of a space available within said interior bone portion for implanting the device (22), said predetermined angle being maintained by at least one adjustment device (44) located on at least one said plate member, said adjustment device being selected from the group consisting of clips, clamps and detents, wherein at least one said plate member is at least partially coated or at least partially impregnated by either a bone anabolic agent for promoting bone growth within an interior portion of a bone wherein the device is implanted, said bone anabolic agent being selected from the group consisting of a parathyroid hormone (PTH) or truncate thereof, in free acid or amide form, anabolic Vitamin D analogs, a low-density lipoprotein receptor related protein 5 (LRP5), an activator of non-genomic estrogen-like signaling (ANGELS), a bone morphogenic protein (BMP), an insulin-like growth factor (IGF), a fibroblast growth factor (FGF), sclerostin, leptin, a prostaglandin, a statin, strontium, a growth hormone, a growth hormone releasing factor (GHRF), hepatocyte growth factor (HGF), calcitonin gene related peptide (CGRP), parathyroid hormone related peptide (PTHrP), transforming growth factor (TGF)-β1 and combinations thereof, or an agent that causes increased expression of an endogenous bone anabolic agent into the blood of a subject within which said device (22) is implanted.

17. The bone implant device (22) of claim 1, wherein at least two said plate members (34, 36, 38) are connected by a hinge member (40, 42) along an edge portion of the plate members thus connected, each said hinge member forming an axis of rotation of the plate members interconnected thereby, said plate members having a shape selected from the group consisting of substantially planar, at least partially curvilinear and open tubular and being formed of a material selected from the group consisting of metals, ceramics, plastics, composites and resins, wherein said plate members are maintained at a predetermined angle to one another, said angle chosen to correspond to an amount and a configuration of a space available within said interior bone portion for implanting the device (22), said predetermined angle being maintained by at least one adjustment device (44) located on at least one said plate member, said adjustment device being selected from the group consisting of clips, clamps and detents, wherein at least one said plate member is at least partially coated or at least partially impregnated by either a bone anabolic agent for promoting bone growth within an interior portion of a bone wherein the device is implanted, said bone anabolic agent being selected from the group consisting of a parathyroid hormone (PTH) or truncate thereof, in free acid or amide form, anabolic Vitamin D analogs, a low-density lipoprotein receptor related protein 5 (LRP5), an activator of non-genomic estrogen-like signaling (ANGELS), a bone morphogenic protein (BMP), an insulin-like growth factor (IGF), a fibroblast growth factor (FGF), sclerostin, leptin, a prostaglandin, a statin, strontium, a growth hormone, a growth hormone releasing factor (GHRF), hepatocyte growth factor (HGF), calcitonin gene related peptide (CGRP), parathyroid hormone related peptide (PTHrP), transforming growth factor (TGF)-β1 and combinations thereof, or an agent that causes increased expression of an endogenous bone anabolic agent into the blood of a subject within which said device (22) is implanted.

18. The bone implant device (22) of claim 1, said plate members (14, 16) being formed of a material selected from the group consisting of metals, ceramics, plastics, composites and resins, wherein said plate members are maintained at a predetermined angle to one another, said angle chosen to correspond to an amount and a configuration of a space available within said interior bone portion for implanting the device (22), said predetermined angle being maintained by at least one adjustment device (44) located on at least one said plate member, said adjustment device being selected from the group consisting of clips, clamps and detents, wherein at least one said plate member is at least partially coated or at least partially impregnated by either a bone anabolic agent for promoting bone growth within an interior portion of a bone wherein the device is implanted, said bone anabolic agent being selected from the group consisting of a parathyroid hormone (PTH) or truncate thereof, in free acid or amide form, anabolic Vitamin D analogs, a low-density lipoprotein receptor related protein 5 (LRP5), an activator of non-genomic estrogen-like signaling (ANGELS), a bone morphogenic protein (BMP), an insulin-like growth factor (IGF), a fibroblast growth factor (FGF), sclerostin, leptin, a prostaglandin, a statin, strontium, a growth hormone, a growth hormone releasing factor (GHRF), hepatocyte growth factor (HGF), calcitonin gene related peptide (CGRP), parathyroid hormone related peptide (PTHrP), transforming growth factor (TGF)-β1 and combinations thereof, or an agent that causes increased expression of an endogenous bone anabolic agent into the blood of a subject within which said device (22) is implanted.

19. The bone implant device (22) of claim 1, said plate members (14, 16) being formed of a material selected from the group consisting of metals, ceramics, plastics, composites and resins, wherein said plate members are maintained at a predetermined angle to one another, said angle chosen to correspond to an amount and a configuration of a space available within said interior bone portion for implanting the device, wherein at least one said plate member is at least partially coated or at least partially impregnated by a material selected from the group consisting of a bone anabolic agent for promoting bone growth within an interior portion of a bone wherein the device is implanted and an agent that causes increased expression of an endogenous bone anabolic agent into the blood of a subject within which said device is implanted.

## Patentansprüche

1. Knochenimplantatsvorrichtung (22), umfassend eine Vielzahl von miteinander verbundenen Plattenelementen (14, 16), die so konfiguriert und eingerichtet sind, dass sie ein zumindest teilweises Einsetzen der Vorrichtung (22) innerhalb eines inneren Knochenabschnitts eines Individuums ermöglichen, wobei der innere Knochenabschnitt eine Knochenmarkhöhle (26) umfasst, die von einer inneren Knochenfläche (30) definiert wird, wobei zumindest ein Plattenelement (14, 16) eine Vielzahl von Öffnungen (24) darin definiert, die so eingerichtet sind, dass sie ein Knochenwachstum durch diese von innerhalb des inneren Knochenabschnitts ermöglichen, um ein Befestigen der Vorrichtung (22) innerhalb des inneren Knochenabschnitts zu unterstützen, wobei nach Installation der Vorrichtung (22) nicht mehr als ungefähr 75 % der inneren Knochenfläche (30) in Berührung mit der Vorrichtung (22) stehen, und **dadurch gekennzeichnet, dass** ein Winkel zwischen zumindest zweien der miteinander verbundenen Plattenelemente (14, 16) einstellbar ist.

2. Implantatsvorrichtung (22) nach Anspruch 1, wobei die Vorrichtung mit zumindest einem Befestigungselement oder mit einem biologisch kompatiblen Haftmittel zumindest teilweise innerhalb des inneren Knochenabschnitts befestigt ist.

3. Implantatsvorrichtung (22) nach Anspruch 1, wobei die Plattenelemente (14, 16) in einem vorab definierten Winkel zueinander gehalten werden, wobei der Winkel so gewählt ist, dass er einer Menge und einer Konfiguration eines Raums entspricht, der innerhalb des inneren Knochenabschnitts zum Implantieren der Vorrichtung verfügbar ist.

4. Implantatsvorrichtung (22) nach Anspruch 3, wobei der vorab definierte Winkel von zumindest einer Einstellvorrichtung (44) gehalten wird, die sich auf zumindest einem Plattenelement befindet, wobei die Einstellvorrichtung (44) aus der Gruppe, bestehend aus Klemmen, Klammern und Rastungen, ausgewählt ist.

5. Implantatsvorrichtung (22) nach Anspruch 1, wobei zumindest ein Plattenelement (14, 16) eine Schlitzöffnung (18, 20) darin definiert, wobei die Schlitzöffnung so konfiguriert und eingerichtet ist, dass sie eine Verriegelungspassung zwischen dem geschlitzten Plattenelement und zumindest einem zusätzlichen Plattenelement ermöglicht.

6. Implantatsvorrichtung (22) nach Anspruch 1, wobei zumindest zwei Plattenelemente (14, 16) über ein Gelenkelement (40, 42) entlang eines Randabschnitts der somit verbundenen Plattenelemente verbunden sind, wobei jedes Gelenkelement (40, 42) eine Drehachse der dadurch verbundenen Plattenelemente bildet.

7. Implantatsvorrichtung (22) nach Anspruch 1, umfassend drei oder mehr Plattenelemente (34, 36, 38), wobei zumindest zwei Plattenelemente über ein Gelenkelement (40, 42) entlang eines Randabschnitts der verbundenen Plattenelemente mit einem benachbarten Element verbunden sind, wobei jedes Gelenkelement eine Drehachse der dadurch miteinander verbundenen Plattenelemente bildet.

8. Implantatsvorrichtung (22) nach Anspruch 1, wobei die Plattenelemente (14, 16) eine Form aufweisen, die aus der Gruppe, bestehend aus im Wesentlichen planar, zumindest teilweise kurvenförmig und offen rohrförmig, ausgewählt ist.

9. Implantatsvorrichtung (22) nach Anspruch 8, wobei das Plattenelement (60) eine offene rohrförmige Form aufweist und mit zumindest einem zusätzlichen Plattenelement (64, 66, 68) versehen ist, das an einem Ende davon an einer Außenfläche des rohrförmigen Plattenelements befestigt ist und das ein zweites, gegenüberliegendes Ende aufweist, das sich vom rohrförmigen Plattenelement nach außen erstreckt.

10. Implantatsvorrichtung (22) nach Anspruch 9, wobei zumindest ein zusätzliches Plattenelement (64, 66, 68), das sich vom rohrförmigen Plattenelement (60) erstreckt, sich in einem einstellbaren Winkel zu einer Außenfläche des rohrförmigen Plattenelements erstreckt.

11. Implantatsvorrichtung (22) nach Anspruch 1, wobei die Plattenelemente (14, 16) aus einem Material gebildet sind, ausgewählt aus der Gruppe, bestehend aus Metallen, Keramiken, Kunststoffen, Verbundstoffen und Harzen.

12. Implantatsvorrichtung (22) nach Anspruch 1, wobei zumindest ein Plattenelement (14, 16) zumindest teilweise mit einem Knochenaufbaumittel beschichtet oder imprägniert ist, um das Knochenwachstum innerhalb des inneren Abschnitts eines Knochens zu fordern, in den die Vorrichtung (22) implantiert ist.

13. Implantatsvorrichtung (22) nach Anspruch 12, wobei das Knochenaufbaumittel aus der Gruppe, bestehend aus einem Parathormin (PTH) oder einer Trunkierung davon, in Form von freier Säure oder Amidform, anabolischen Vitamin-D-Analogen, einem Low Density Lipoprotein Receptor-Related Protein 5 (LRP5), einem Aktivator der nicht-genomischen östrogenähnlichen Signalgebung (MANGELS), einem knochenmorphogenetischem Protein (BMP), einem insulinähnlichen Wachstumsfaktor (IGF), einern Fibrobtastenwachstumsfaktor (FGF), Sclerostin, Leptin, einem Prostaglandin, einem Statin, Strontium, einem Wachstumshormon, einem Wachstumshormonfreisetzungsfaktor (GHRF), Hepatozytenwachstumsfaktor (HGF), Calcitonin Gene-Related Peptid (CGRP), Parathyroid Hormone-Related Peptide (PTHrP), transformierendem Wachstumsfaktor-(TGF)-β1 und Kombinationen davon, ausgewählt ist.

14. Implantatsvorrichtung (22) nach Anspruch 12, wobei das Knochenaurfbaumittel aus der Gruppe, bestehend aus PTH[1-30], PTH[1-31], PTH[1-32], PTH[1-33] und PTH[1-34], in Form von freier Säure oder Amidform, sowie Kombinationen davon, ausgewählt ist.

15. Implatatsvorrichtung (22) nach Anspruch 1, wobei zumindest ein Plattenelement (14, 16) zumindest teilweise mit einem Mittel beschichtet oder imprägniert ist, das eine erhöhte Expression eines endogenen Knochenaufbaumittels in das Blut eines Individuums bewirkt, in das die Vorrichtung (22) implantiert ist.

16. Knochmimplantatsvorrichtung (22) nach Anspruch 1, wobei zumindest ein Plattenelement (14, 16) eine Schlitzöffnung (18, 20) darin definiert, wobei die Schlitzöffnung so konfiguriert und eingerichtet ist, dass sie eine Verriegelungspassung zwischen dem geschlitzten Plattenelement und zumindest einem zusätzlichen Plattenelement ermöglicht, wobei die Plattenelemente eine Form aufweisen, die aus der Gruppe, bestehend aus im Wesentlichen planar, zumindest teilweise kurvenförmig und offen rohrförmig, ausgewählt ist und aus einem Material gebildet sind, das aus der Gruppe, bestehend aus Metallen, Keramiken, Kunststoffen, Verbundstoffen und Harzen, ausgewählt ist, wobei die Plattenelemente in einem vorab definierten Winkel zueinander gehalten werden, wobei der Winkel so gewählt ist, dass er einer Menge und einer Konfiguration eines Raums entspricht, der innerhalb des inneren Knochenabschnitts zum Implantieren der Vorrichtung (22) verfügbar ist, wobei der vorab definierte Winkel von zumindest einer Einstellvorrichtung (44) gehalten wird, die sich auf zumindest einem Plattenelement befindet, wobei die Einstellvorrichtung aus der Gruppe, bestehend aus Klemmen, Klammern und Rastungen, ausgewählt ist, wobei zumindest ein Plattenelement zumindest teilweise mit entweder einem Knochenaufbaumittel beschichtet oder imprägniert ist, um das Knochenwachstum innerhalb eines inneren Abschnitts eines Knochens zu fördern, in den die Vorrichtung implantiert ist, wobei das Knochenautbaumittel aus der Gruppe, bestehend aus einem Parathormin (PTH) oder einer Trunkierung davon, in Form von freier Säure oder Amidform, anabolischen Vitamin-D-Analogen, einem Low Density Lipoprotein Receptor-Related Protein 5 (LRP5), einem Aktivator der nicht-genomischen östrogenähnlichen Signalgebung (ANGELS), einem knochenmorphogenetischem Protein (BMP), einem insulinähnlichen Wachstumsfaktor (IGF), einem Fibroblastenwachstumsfaktor (FGF), Sclerostin, Leptin, einem Prostaglandin, einem Statin, Strontium, einem Wachstumshormon, einem Wachstumshomtonfreisetzungsfaktor (GHRF), Hepatozytenwachstumsfaktor (HGF), Calcitonin Gene-Related Peptid (CGRP), Parathyroid Hormone-Related Peptide (PTHrP), transformierendem Wachstumsfaktor-(TGF)-β1 und Kombinationen davon, ausgewählt ist, oder mit einem Mittel, das eine erhöhte Expression eines endogenen Knochenaufbaumittels in das Blut eines Individuums bewirkt, in das die Vorrichtung (22) implantiert ist.

17. Knochenimplantatsvorrichtung (22) nach Anspruch 1, wobei zumindest zwei Plattenelemente (34, 36, 38) über ein Gelenkelement (40, 42) entlang eines Randabschnitts der somit verbundenen Plattenelemente verbunden sind, wobei jedes Gelenkelement eine Drehachse der dadurch miteinander verbundenen Plattenelemente bildet, wobei die Plattenelemente eine Form aufweisen, die aus der Gruppe, bestehend aus im Wesentlichen planar, zumindest teilweise kurvenförmig und offen rohrförmig, ausgewählt ist und aus einem Material gebildet sind, das aus der Gruppe, bestehend aus Metallen, Keramiken, Kunststoffen, Verbundstoffen und Harzen, ausgewählt ist, wobei die Plattenelemente in einem vorab definierten Winkel zueinander gehalten werden, wobei der Winkel so gewählt ist, dass er einer Menge und einer Konfiguration eines Raums entspricht, der innerhalb des inneren Knochenabschnitts zum Implantieren der Vorrichtung (22) verfügbar ist, wobei der vorab definierte Winkel von zumindest einer Einstellvorrichtung (44) gehalten wird, die sich auf zumindest einem Plattenelement befindet, wobei die Einstellvorrichtung aus der Gruppe, bestehend aus Klemmen, Klammern und Rastungen, ausgewählt ist, wobei zumindest ein Plattenelement zumindest teilweise mit entweder einem Knochenaufbaumittel beschichtet oder imprägniert ist, um das Knochenwachstum innerhalb eines inneren Abschnitts eines Knochens zu fördern, in den die Vorrichtung implantiert ist, wobei das Knochenaufbaumittel aus der Gruppe, bestehend aus einem Parathormin (PTH) oder einer Trunkierung davon, in Form von freier Säure oder Amidform, anabolischen Vitamin-D-Analogen, einem Low Density Lipoprotein Receptor-Related Protein 5 (LRP5), einem Aktivator der nicht-genomischen östrogenähnlichen Signalgebung (ANGELS), einem knochenmorphogenetischem Protein (BMP), einem insulinähnlichen Wachstumsfaktor (IGF), einem Fibroblastenwachstumsfaktor (FGF), Sclerostin, Leptin, einem Prostaglandin, einem Statin, Strontium, einem Wachstumshormon, einem Wachstumshormonfreisetzungsfaktor (GHRF), Hepatozytenwachstumsfaktor (HGF), Calcitonin Gene-Related Peptid (CGRP), Parathyroid Hormone-Related Peptide (PTHrP), transformierendem Wachstumsfaktor-(TGF)-β1 und Kombinationen davon, ausgewählt ist, oder mit einem Mittel, das eine erhöhte Expression eines endogenen Knochenaufbaumittels in das Blut eines Individuums bewirkt, in das die Vorrichtung (22) implantiert ist.

18. Knochenimptantatsvorrichtung (22) nach Anspruch 1, wobei die Plattenelemente (14, 16) aus einem Material gebildet sind, ausgewählt aus der Gruppe, bestehend aus Metallen, Keramiken, Kunststoffen, Verbundstoffen und Harzen, wobei die Plattenelemente in einem vorab definierten Winkel zueinander gehalten werden, wobei der Winkel so gewählt ist, dass er einer Menge und einer Konfiguration eines Raums entspricht, der innerhalb des inneren Knochenabschnitts zum Implantieren der Vorrichtung (22) verfügbar ist, wobei der vorab definierte Winkel von zumindest einer Einstellvorrichtung (44) gehalten wird, die sich auf zumindest einem Plattenelement befindet, wobei die Einstellvorrichtung aus der Gruppe, bestehend aus Klemmen, Klammern und Rastungen, ausgewählt ist, wobei zumindest ein Plattenelement zumindest teilweise mit entweder einem Knochenaufbaumittel beschichtet oder imprägniert ist, um das Knochenwachstum innerhalb eines inneren Abschnitts eines Knochens zu fördern, in den die Vorrichtung implantiert ist, wobei das Knochenaufbaumittel aus der Gruppe, bestehend aus einem Parathormin (PTH) oder einer Trunkierung davon, in Form von freier Säure oder Amidform, anabolischen Vitamin-D-Analogen, einem Low Density Lipoprotein Receptor-Related Protein 5 (LRP5), einem Aktivator der nicht-genomischen östrogenähnlichen Signalgebung (ANGELS), einem knochenmorphogenetischen Protein (BMP), einem insulinähnlichen Wachstumsfaktor (IGF), einem Fibroblastenwachstumsfaktor (FGF), Sclerostin, Leptin, einem Prostaglandin, einem Statin, Strontium, einem Wachstumshormon, einem Wachstumshormonfreisetzungsfaktor (GHRF), Hepatozytenwachstumsfaktor (HGF), Calcitonin Gene-Related Peptid (CGRP), Parathyroid Hormone-Related Peptide (PTHrP), transformierendem Wachstumsfaktor-(TGF)-β1 und Kombinationen davon, ausgewählt ist, oder mit einem Mittel, das eine erhöhte Expression eines endogenen Knochenaufbaumittels in das Blut eines Individuums bewirkt, in das die Vorrichtung (22) implantiert ist.

19. Knochenimplantatsvorrichtung (22) nach Anspruch 1, wobei die Plattenelemente (14, 16) aus einem Material gebildet sind, ausgewählt aus der Gruppe, bestehend aus Metallen, Keramiken, Kunststoffen, Verbundstoffen und Harzen, wobei die Plattenelemente in einem vorab definierten Winkel zueinander gehalten werden, wobei der Winkel so gewählt ist, dass er einer Menge und einer Konfiguration eines Raums entspricht, der innerhalb des inneren Knochenabschnitts zum Implantieren der Vorrichtung verfügbar ist, wobei zumindest ein Plattenelement zumindest teilweise mit einem Material beschichtet oder imprägniert ist, das aus der Gruppe, bestehend aus einem Knochenaufbaumittel zum Fördem des Knochenwachstums innerhalb eines inneren Abschnitts eines Knochens, in den die Vorrichtung implantiert ist, und einem Mittel, das eine erhöhte Expression eines endogenen Knochenaufbaumittels in das Blut eines Individuums bewirkt, in das die Vorrichtung implantiert ist, ausgewählt ist.

## Revendications

1. Dispositif d'implant osseux (22) comprenant une pluralité d'éléments de plaque interconnectés (14, 16) configurés et adaptés pour permettre au moins une insertion partielle du dispositif (22) au sein d'une partie osseuse interne d'un sujet, ladite partie osseuse interne comprenant une cavité de moelle osseuse (26) définie par une surface osseuse interne (30), au moins l'un desdits éléments de plaque (14, 16) définissant une pluralité d'ouvertures (24) dans celui-ci qui sont adaptées pour permettre une croissance osseuse à travers celles-ci à partir de l'intérieur de ladite partie osseuse interne, afin de faciliter la sécurisation du dispositif (22) au sein de la partie osseuse interne, où, après l'installation du dispositif (22), au maximum environ 75 % de la surface osseuse interne (30) est en contact avec le dispositif (22), et **caractérisé en ce qu'**un angle entre au moins deux desdits éléments de plaque interconnectés (14, 16) est ajustable.

2. Dispositif d'implant (22) selon la revendication 1, où le dispositif est au moins partiellement sécurisé au sein de ladite partie osseuse interne avec au moins un élément de fixation ou avec un adhésif biocompatible.

3. Dispositif d'implant (22) selon la revendication 1, dans lequel lesdits éléments de plaque (14, 16) sont maintenus à un angle prédéterminé les uns par rapport aux autres, ledit angle étant choisi pour correspondre à une quantité et à une configuration d'un espace disponible au sein de ladite partie osseuse interne afin d'implanter le dispositif.

4. Dispositif d'implant (22) selon la revendication 3, dans lequel ledit angle prédétermine est maintenu par au moins un dispositif d'ajustement (44) situé sur au moins un desdits éléments de plaque, ledit dispositif d'ajustement (44) étant sélectionné dans le groupe consistant en des pinces, des colliers de serrage et des crans.

5. Dispositif d'implant (22) selon la revendication 1, dans lequel au moins un desdits éléments de plaque (14, 16) définit une ouverture fendue (18, 20) dans celui-ci, ladite ouverture fendue étant configurée et adaptée pour permettre un ajustement par verrouillage entre ledit élément de plaque fendu et au moins un élément de plaque supplémentaire.

6. Dispositif d'implant (22) selon la revendication 1, dans lequel au moins deux desdits éléments de plaque (14, 16) sont connectés par un élément de charnière (40, 42) le long d'une partie de bord des éléments de plaque ainsi connectés, chacun desdits éléments de charnière (40, 42) formant un axe de rotation des éléments de plaque connectés par celui-ci.

7. Dispositif d'implant (22) selon la revendication 1, comprenant au moins trois desdits éléments de plaque (34, 36, 38), où au moins deux desdits éléments de plaque sont connectés à un élément adjacent par un élément de charnière (40, 42) le long d'une partie de bord des éléments de plaque connectés, chacun desdits éléments de charnière formant un axe de rotation des éléments de plaque interconnectés par celui-ci.

8. Dispositif d'implant (22) selon la revendication 1, dans lequel lesdits éléments de plaque (14, 16) ont une forme qui est sélectionnée dans le groupe consistant en une forme essentiellement plane, au moins partiellement curvilinéaire, et tubulaire ouverte.

9. Dispositif d'implant (22) selon la revendication 8, dans lequel ledit élément de plaque (60) possède une forme tubulaire ouverte et est mis à disposition avec au moins un élément de plaque supplémentaire (64, 66, 68) qui est fixé, au niveau d'une extrémité de celui-ci, à une surface externe dudit élément de plaque tubulaire, et ayant une seconde extrémité opposée qui s'étend vers l'extérieur à partir dudit élément de plaque tubulaire.

10. Dispositif d'implant (22) selon la revendication 9, dans lequel au moins un desdits éléments de plaque supplémentaires (64, 66, 68) s'étendant à partir dudit élément de plaque tubulaire (60) s'étend selon un angle ajustable vers une surface externe dudit élément de plaque tubulaire.

11. Dispositif d'implant (22) selon la revendication 1, dans lequel lesdits éléments de plaque (14, 16) sont formés d'un matériau sélectionné dans le groupe consistant en des métaux, des céramiques, des plastiques, des composites et des résines.

12. Dispositif d'implant (22) selon la revendication 1, dans lequel au moins un desdits éléments de plaque (14, 16) est au moins partiellement revêtu ou au moins partiellement imprégné d'un agent anabolisant osseux afin de favoriser la croissance osseuse au sein de la partie interne d'un os dans lequel le dispositif (22) est implanté.

13. Dispositif d'implant (22) selon la revendication 12, dans lequel l'agent anabolisant osseux est sélectionné dans le groupe consistant en une hormone parathyroïdienne (PTH) ou une version tronquée de celle-ci, sous une forme acide libre ou amide, des analogues de vitamine D anabolisants, une protéine 5 apparentée au récepteur des lipoprotéines de basse densité (LRP5), un activateur de signaux de type oestrogène non génomique (ANGELS), une protéine morphogénétique osseuse (BMP), un facteur de croissance analogue à l'insuline (IGF), un facteur de croissance des fibroblastes (FGF), la sclérostine, la leptine, une prostaglandine, une statine, le strontium, une hormone de croissance, un facteur de libération de l'hormone de croissance (GHRF), le facteur de croissance des hépatocytes (HGF), le peptide apparenté au gène de la calcitonine (CGRP), le peptide apparenté à l'hormone parathyroïdienne (PTHrP), le facteur de croissance transformant (TGF)-β1 et des combinaisons de ceux-ci.

14. Dispositif d'implant (22) selon la revendication 12, dans lequel l'agent anabolisant osseux est sélectionné dans le groupe consistant en la PTH[1-30], la PTII[1-31], la PTH[1-32], la PTH[1-33], et la PTH[1-34], sous la forme acide libre ou amide, et des combinaisons de celles-ci.

15. Dispositif d'implant (22) selon la revendication 1, dans lequel au moins un desdits éléments de plaque (14, 16) est au moins partiellement revêtu ou au moins partiellement imprégné d'un agent qui entraîne une expression augmentée d'un agent anabolisant osseux endogène dans le sang d'un sujet chez qui ledit dispositif (22) est implanté.

16. Dispositif d'implant osseux (22) selon la revendication 1, dans lequel au moins un desdits éléments de plaque (14, 16) définit une ouverture fendue (18, 20), ladite ouverture fendue étant configurée et adaptée pour permettre un ajustement par verrouillage entre ledit élément de plaque fendu et au moins un élément de plaque supplémentaire, lesdits éléments de plaque ayant une forme sélectionnée dans le groupe consistant en une forme essentiellement plane, au moins partiellement curvilinéaire, et tubulaire ouverte, et étant formés d'un matériau sélectionné dans le groupe consistant en des métaux, des céramiques, des plastiques, des composites et des résines, où lesdits éléments de plaque sont maintenus à un angle prédéterminé les uns par rapport aux autres, ledit angle étant choisi pour correspondre à une quantité et à une configuration d'un espace disponible au sein de ladite partie osseuse interne afin d'implanter le dispositif (22), ledit angle prédéterminé étant maintenu par au moins un dispositif d'ajustement (44) situé sur au moins un desdits éléments de plaque, ledit dispositif d'ajustement étant sélectionné dans le groupe consistant en des pinces, des colliers de serrage et des crans, où au moins un desdits éléments de plaque est au moins partiellement revêtu ou au moins partiellement imprégné de soit un agent anabolisant osseux afin de favoriser la croissance osseuse au sein d'une partie interne d'un os dans lequel le dispositif est implanté, ledit agent anabolisant osseux étant sélectionné dans le groupe consistant en une hormone parathyroïdienne (PTH) ou une version tronquée de celle-ci, sous une forme acide libre ou amide, des analogues de vitamine D anabolisants, une protéine 5 apparentée au récepteur des lipoprotéines de basse densité (LRP5), un activateur de signaux de type oestrogène non génomique (ANGELS), une protéine morphogénétique osseuse (BMP), un facteur de croissance analogue à l'insuline (IGF), un facteur de croissance des fibroblastes (FGF), la sclérostine, la leptine, une prostaglandine, une statine, le strontium, une hormone de croissance, un facteur de libération de l'hormone de croissance (GHRF), le facteur de croissance des hépatocytes (HGF), le peptide apparenté au gène de la calcitonine (CGRP), le peptide apparenté à l'hormone parathyroïdienne (PTHrP), le facteur de croissance transformant (TGF)-β1 et des combinaisons de ceux-ci, ou d'un agent qui entraîne une expression augmentée d'un agent anabolisant osseux endogène dans le sang d'un sujet chez qui ledit dispositif (22) est implanté.

17. Dispositif d'implant osseux (22) selon la revendication 1, dans lequel au moins deux desdits éléments de plaque (34, 36, 38) sont connectés par un élément de charnière (40, 42) le long d'une partie de bord des éléments de plaque ainsi connectés, chacun desdits éléments de charnière formant un axe de rotation des éléments de plaque interconnectés par celui-ci, lesdits éléments de plaque ayant une forme sélectionnée dans le groupe consistant en une forme essentiellement plane, au moins partiellement curvilinéaire, et tubulaire ouverte, et étant formés d'un matériau sélectionné dans le groupe consistant en des métaux, des céramiques, des plastiques, des composites et des résines, où lesdits éléments de plaque sont maintenus à un angle prédéterminé les uns par rapport aux autres, ledit angle étant choisi pour correspondre à une quantité et à une configuration d'un espace disponible au sein de ladite partie osseuse interne afin d'implanter le dispositif (22), ledit angle prédéterminé étant maintenu par au moins un dispositif d'ajustement (44) situé sur au moins un desdits éléments de plaque, ledit dispositif d'ajustement étant sélectionné dans le groupe consistant en des pinces, des colliers de serrage et des crans, où au moins un desdits éléments de plaque est au moins partiellement revêtu ou au moins partiellement imprégné de soit un agent anabolisant osseux afin de favoriser la croissance osseuse au sein d'une partie interne d'un os dans lequel le dispositif est implanté, ledit agent anabolisant osseux étant sélectionné dans le groupe consistant en une hormone parathyroïdienne (PTH) ou une version tronquée de celle-ci, sous une forme acide libre ou amide, des analogues de vitamine D anabolisants, une protéine 5 apparentée au récepteur des lipoprotéines de basse densité (LRP5), un activateur de signaux de type oestrogène non génomique (ANGELS), une protéine morphogénétique osseuse (BMP), un facteur de croissance analogue à l'insuline (IGF), un facteur de croissance des fibroblastes (FGF), la sclérostine, la leptine, une prostaglandine, une statine, le strontium, une hormone de croissance, un facteur de libération de l'hormone de croissance (GHRF), le facteur de croissance des hépatocytes (HGF), le peptide apparenté au gène de la calcitonine (CGRP), le peptide apparenté à l'hormone parathyroïdienne (PTHrP), le facteur de croissance transformant (TGF)-β1 et des combinaisons de ceux-ci, ou d'un agent qui entraîne une expression augmentée d'un agent anabolisant osseux endogène dans le sang d'un sujet chez qui ledit dispositif (22) est implanté.

18. Dispositif d'implant osseux (22) selon la revendication 1, lesdits éléments de plaque (14, 16) étant formés d'un matériau sélectionné dans le groupe consistant en des métaux, des céramiques, des plastiques, des composites et des résines, où lesdits éléments de plaque sont maintenus à un angle prédéterminé les uns par rapport aux autres, ledit angle étant choisi pour correspondre à une quantité et à une configuration d'un espace disponible au sein de ladite partie osseuse interne afin d'implanter le dispositif (22), ledit angle prédéterminé étant maintenu par au moins un dispositif d'ajustement (44) situé sur au moins un desdits éléments de plaque, ledit dispositif d'ajustement étant sélectionné dans le groupe consistant en des pinces, des colliers de serrage et des crans, où au moins un desdits éléments de plaque est au moins partiellement revêtu ou au moins partiellement imprégné de soit un agent anabolisant osseux afin de favoriser la croissance osseuse au sein d'une partie interne d'un os dans lequel le dispositif est implanté, ledit agent anabolisant osseux étant sélectionné dans le groupe consistant en une hormone parathyroïdienne (PTH) ou une version tronquée de celle-ci, sous une forme acide libre ou amide, des analogues de vitamine D anabolisants, une protéine 5 apparentée au récepteur des lipoprotéines de basse densité (LRP5), un activateur de signaux de type oestrogène non génomique (ANGELS), une protéine morphogénétique osseuse (BMP), un facteur de croissance analogue à l'insuline (IGF), un facteur de croissance des fibroblastes (FGF), la sclérostine, la leptine, une prostaglandine, une statine, le strontium, une hormone de croissance, un facteur de libération de l'hormone de croissance (GHRF), le facteur de croissance des hépatocytes (HGF), le peptide apparenté au gène de la calcitonine (CGRP), le peptide apparenté à l'hormone parathyroïdienne (PTHrP), le facteur de croissance transformant (TGF)-β1 et des combinaisons de ceux-ci, ou d'un agent qui entraîne une expression augmentée d'un agent anabolisant osseux endogène dans le sang d'un sujet chez qui ledit dispositif (22) est implanté.

19. Dispositif d'implant osseux (22) selon la revendication 1, lesdits éléments de plaque (14, 16) étant formés d'un matériau sélectionné dans le groupe consistant en des métaux, des céramiques, des plastiques, des composites et des résines, où lesdits éléments de plaque sont maintenus à un angle prédéterminé les uns par rapport aux autres, ledit angle étant choisi pour correspondre à une quantité et à une configuration d'un espace disponible au sein de ladite partie osseuse interne afin d'implanter le dispositif, où au moins un desdits éléments de plaque est au moins partiellement revêtu ou au moins partiellement imprégné d'un matériau sélectionné dans le groupe consistant en un agent anabolisant osseux afin de favoriser la croissance osseuse au sein d'une partie interne d'un os dans lequel le dispositif est implanté, et un agent qui entraîne une expression augmentée d'un agent anabolisant osseux endogène dans le sang d'un sujet chez qui ledit dispositif est implanté.
